# EUROPEAN PATENT APPLICATION

(11) **EP 3 260 123 A1**
(43) Date of publication of application: **27.12.2017**
(21) Application number: 17180935.3
(22) Date of filing: 06.11.2009
(51) Int. Cl.: A61K 31/7088, A61K 38/00, A61P 13/12

(54) **ROLE OF SOLUBLE UPAR IN THE PATHOGENESIS OF PROTEINURIC KIDNEY DISEASE**

(30) Priority: 06.11.2008 US 111873 P
(62) Divisional of application: 09825472.5
(71) Applicant: University of Miami, Miami, FL 33136 (US); The General Hospital Corporation d/b/a Massachusetts General Hospital, Boston, MA 02114 (US)
(72) Inventor: Reiser, Jochen, Miami, FL Florida 33143 (US)
(74) Representative: Inspicos P/S

(57) **Abstract**

Compositions which specifically block urokinase receptor (uPAR) in podocytes; compositions which modulate or block soluble urokinase receptor (suPAR); compositions which modulate pathways in which uP AR is associated with, protect against renal diseases or disorders. Methods of treatment *in vivo* involve use of one or more compositions.

## Description

### CROSS-REFERENCE TO RELATED APPLICATIONS

The present application claims the priority of U.S. provisional patent application No. 61/111,873 filed November 6, 2008 which is incorporated herein by reference in its entirety.

### FIELD OF THE INVENTION

Embodiments of the invention comprise compositions which modulate expression, function, activity of urokinase receptor (uPAR) in kidneys.

### BACKGROUND

The fact that glomerular kidney diseases after renal transplantation can reoccur within hours after surgery represent a persistent mystery in medicine that harbors enormous challenges. The last few decades witnessed much progress in podocyte biology, however, the exact pathogenesis underlying most proteinuric kidney diseases is still far from clear. This is true with the focal segmental glomerulosclerosis (FSGS). Though mutations to certain molecules including podocin, nephrin, α-actinin, TRPC6, have been claimed responsible for some familial form of FSGS, little is known about the pathogenesis of acquired form of FSGS. FSGS frequently leads to end-stage renal failure, and significantly recurring in 30-60% of transplanted kidneys.

### SUMMARY

This Summary is provided to present a summary of the invention to briefly indicate the nature and substance of the invention. It is submitted with the understanding that it will not be used to interpret or limit the scope or meaning of the claims.

Embodiments of the invention are directed to compositions for the treatment renal diseases or disorders, such as for example, proteinuria.

Other aspects are described *infra.*

### BRIEF DESCRIPTION OF THE DRAWINGS

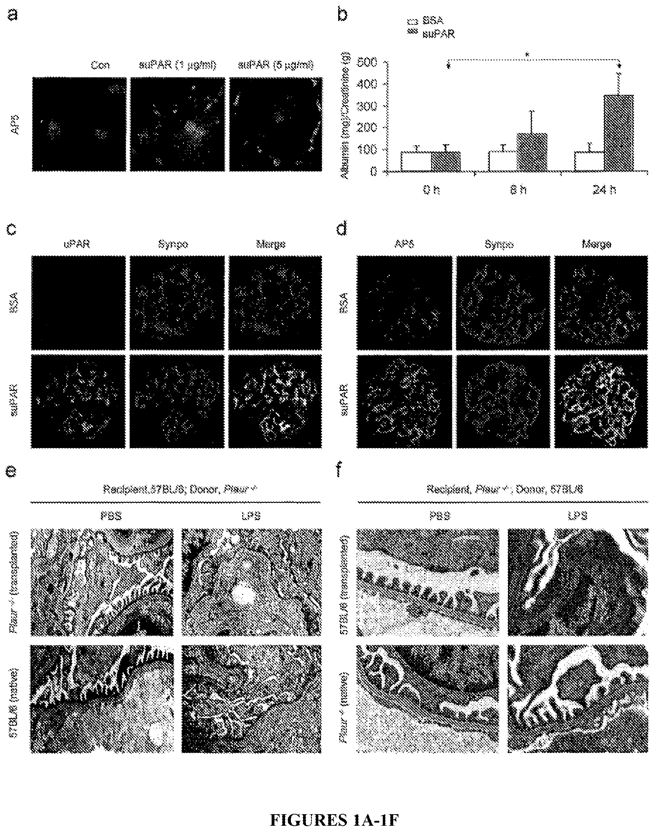

Figures 1A to 1F show that suPAR activates αvβ3 integrin and induce foot process effacement and proteinuria in mice. Figure 1A: suPAR protein activates αvβ3 integrin on cultured podocytes. Fully differentiated mouse podocytes were incubated with suPAR protein at 1-5 µg/ml for 24 h at 37°C, and stained with AP5 antibody. Figure 1B: Injection of suPAR into *Plaur* ^{-/-} mice induces proteinuria. suPAR protein was injected through tail vein into *Plaut* ^{-/-} mice at 1 mg/kg (n = 6), control mice received the same amount of BSA (*n* = 6). Urine was collected before and 8 h, 24 h after injection for Braford assay. ^{*} *P*<O.03 for 24 h after suPAR injection versus 0 h, and versus all BSA groups. Figure 1C: suPAR deposited into podocytes after suPAR injection. Synpo, synaptopodin. Figure 1D: suPAR injection activated αᵥβ₃ integrin in podocytes. Figures 1E, 1F: Kidney cross-transplantation. Figure 1E: The right kidneys from *Plaur*^{*-*/*-*} mice were transplanted into 57BL/6 mice, and then treated with LPS (right panel) or PBS (left panel). Figure 1F: The right kidneys from 57BL/6 mice were transplanted into *Plaur* ^{-/-} mice, and then treated with LPS or PBS.

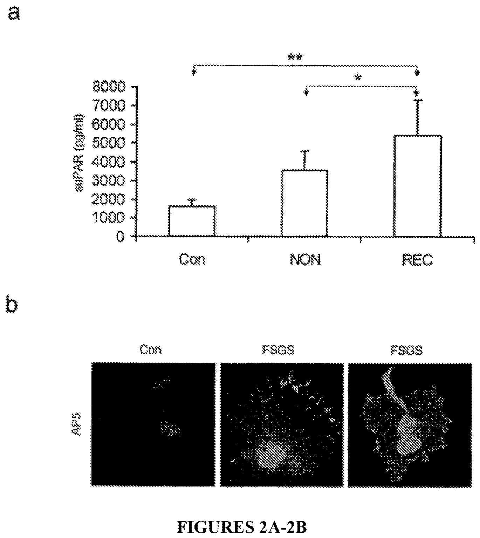

Figures 2A-2B: show that FSGS recurrent patient sera have high level of suPAR, and activate αᵥβ₃ integrin in podocytes. Figure 2A: FSGS recurrent patients had higher levels of serum suPAR than non-recurrent patients and than normal subjects. Figure 2B: Recurrent FSGS sera activated αvβ3 integrin in cultured podocytes.

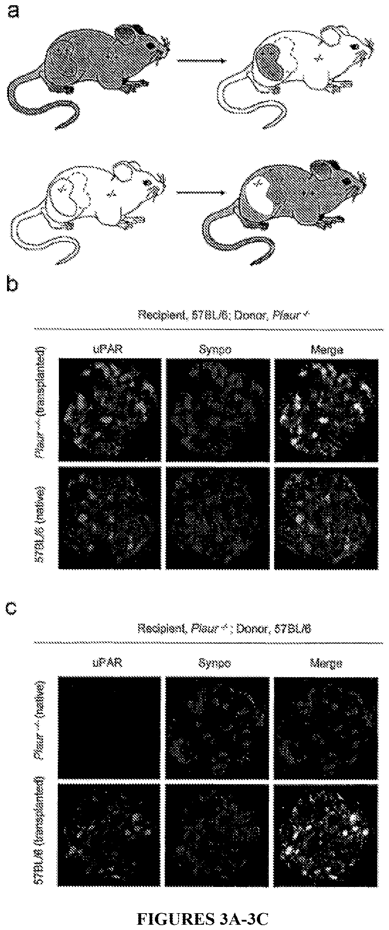

Figures 3A-3C: Kidney cross-transplantation and suPAR in podocytes. Figure 3A is a schematic illustration of cross-transplantation. Figure 3B: suPAR deposited into podocytes in the transplanted *Plaur* ^{-/-} mice. Immunostaining was performed on kidney sections after transplantation and LPS treatment. Synpo, synaptopodin. Figure 3C: shows that in LPS treated *Plaur*^{*-*/*-*} mice, increased expression was found in the transplanted 57BL/6 kidney, but not any uPAR in podocytes of native *Plaur*^{*-*/*-*} kidney.

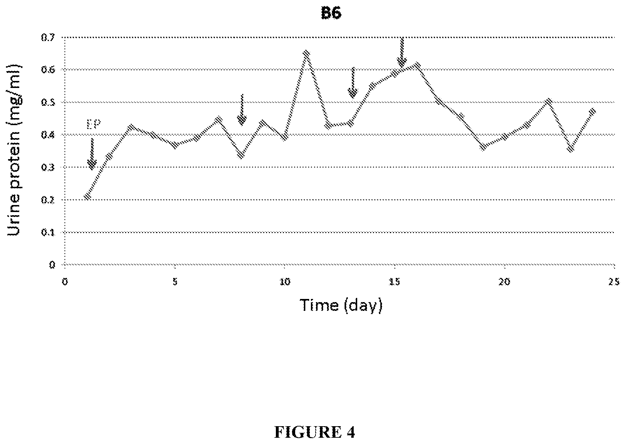

Figure 4: is a graph showing subcutaneous gene delivery of suPAR by electroporation (EP) into WT B6 mice induces proteinuria (arrow indicates when EP was done).

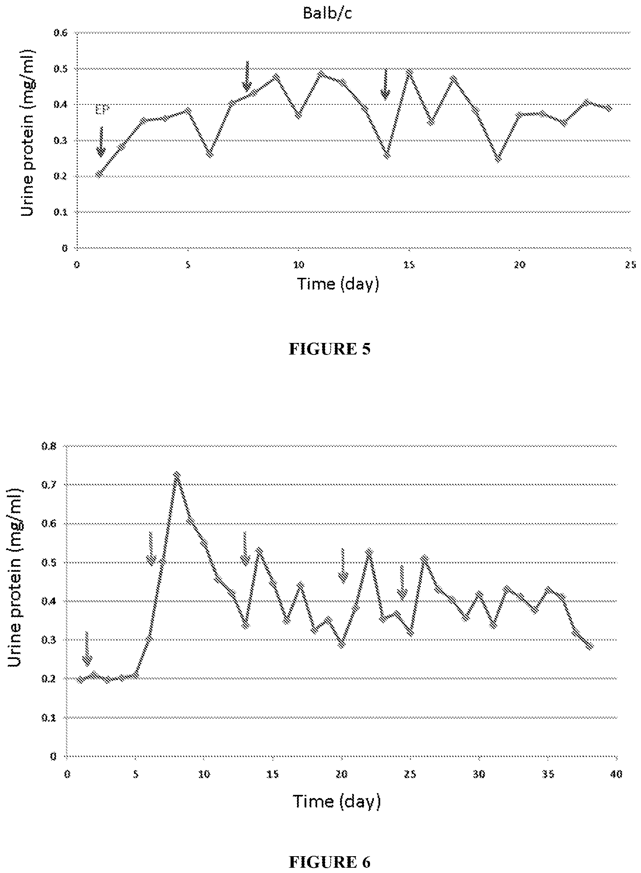

Figure 5 is a graph showing subcutaneous gene delivery of suPAR by electroporation (EP) into WT Balb/c mice induces proteinuria (arrow indicates when EP was done). Both Figures 4 and 5 show the role of soluble urokinase receptor in the development of proteinuria and glomerular kidney disease. Figures 4 and 5 show the expression of GFP-tagged soluble uPAR in the subcutaneous tissue of mice. From there, soluble uPAR is produced and secreted into the blood stream, resulting in proteinuria.
Figure 6 is a graph showing subcutaneous gene delivery of suPAR by electroporation (EP) into uPAR knockout mice induces proteinuria (arrow indicates when EP was done). 24 h after suPAR-GFP gene delivery (EP) into uPAR knockout mouse subcutaneous skin (SC), the mice were examined under IF microscopy. The results showed that GFP-tagged suPAR was expressed in subcutaneous areas of uPAR knockout mice.

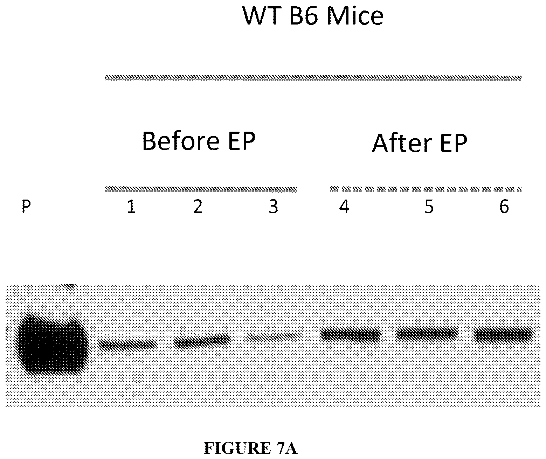

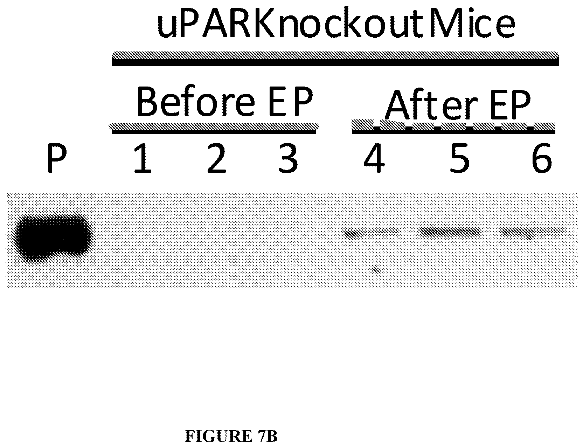

Figures 7A-7B show serum expression of soluble uPAR after gene electroporation in WT and uPAR KO mice. Figure 7A is an immunoblot showing that gene delivery of suPAR by EP into skin increases circulating suPAR level in WT mice. The levels were measured 24 hours after electroporation of the suPAR gene vector into skin. Figure 7B shows gene delivery of suPAR by EP into skin generates circulating suPAR in uPAR knockout mice. The levels were measured 24 hours after electroporation of the suPAR gene vector into skin.

### DETAILED DESCRIPTION

Embodiments of the present invention relates to discoveries involving agents which modulate and/or inhibit urokinase receptor (uPAR), in particular, soluble urokinase receptor (suPAR). Accordingly, the methods of the present invention can be used to treat kidney diseases or disorders, such as, for example, those characterized by proteinuria.

Several aspects of the invention are described below with reference to example applications for illustration. It should be understood that numerous specific details, relationships, and methods are set forth to provide a full understanding of the invention. One having ordinary skill in the relevant art, however, will readily recognize that the invention can be practiced without one or more of the specific details or with other methods. The present invention is not limited by the illustrated ordering of acts or events, as some acts may occur in different orders and/or concurrently with other acts or events. Furthermore, not all illustrated acts or events are required to implement a methodology in accordance with the present invention.

All genes, gene names, and gene products disclosed herein are intended to correspond to homologs from any species for which the compositions and methods disclosed herein are applicable. Thus, the terms include, but are not limited to genes and gene products from humans and mice. It is understood that when a gene or gene product from a particular species is disclosed, this disclosure is intended to be exemplary only, and is not to be interpreted as a limitation unless the context in which it appears clearly indicates. Thus, for example, for the genes disclosed herein, which in some embodiments relate to mammalian nucleic acid and amino acid sequences are intended to encompass homologous and/or orthologous genes and gene products from other animals including, but not limited to other mammals, fish, amphibians, reptiles, and birds. In preferred embodiments, the genes or nucleic acid sequences are human.

### Definitions

The terminology used herein is for the purpose of describing particular embodiments only and is not intended to be limiting of the invention. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

As used herein, the term "safe and effective amount" or "therapeutic amount" refers to the quantity of a component which is sufficient to yield a desired therapeutic response without undue adverse side effects (such as toxicity, irritation, or allergic response) commensurate with a reasonable benefit/risk ratio when used in the manner of this invention. By "therapeutically effective amount" is meant an amount of a compound of the present invention effective to yield the desired therapeutic response. The specific safe and effective amount or therapeutically effective amount will vary with such factors as the particular condition being treated, the physical condition of the patient, the type of mammal or animal being treated, the duration of the treatment, the nature of concurrent therapy (if any), and the specific formulations employed and the structure of the compounds or its derivatives.

As used herein "proteinuria" refers to any amount of protein passing through a podocyte that has suffered podocyte damage or through a podocyte mediated barrier that normally would not allow for any protein passage. In an *in vivo* system the term "proteinuria" refers to the presence of excessive amounts of serum protein in the urine. Proteinuria is a characteristic symptom of either renal (kidney), urinary, pancreatic distress, nephrotic syndromes (i.e., proteinuria larger than 3.5 grams per day), eclampsia, toxic lesions of kidneys, and it is frequently a symptom of diabetes mellitus. With severe proteinuria general hypoproteinemia can develop and it results in diminished oncotic pressure (ascites, edema, hydrothorax).

The phrase "specifically binds to", "is specific for" or "specifically immunoreactive with", when referring to an antibody refers to a binding reaction which is determinative of the presence of the protein in the presence of a heterogeneous population of proteins and other biologics. Thus, under designated immunoassay conditions, the specified antibodies bind to a particular protein and do not bind in a significant amount to other proteins present in the sample. Specific binding to a protein under such conditions may require an antibody that is selected for its specificity for a particular protein.

As used herein, the term "aptamer" or "selected nucleic acid binding species" shall include non-modified or chemically modified RNA or DNA. The method of selection may be by, but is not limited to, affinity chromatography and the method of amplification by reverse transcription (RT) or polymerase chain reaction (PCR).

As used herein, "modulation" means either an increase (stimulation) or a decrease (inhibition) in the expression, *in vivo* amounts of a gene. This includes any amounts *in vivo,* functions and the like as compared to normal controls. The term includes, for example, increased, enhanced, increased, agonized, promoted, decreased, reduced, suppressed blocked, or antagonized. Modulation can increase activity or amounts more than 1-fold, 2-fold, 3-fold, 5-fold, 10-fold, 100-fold, etc., over baseline values. Modulation can also decrease its activity or amounts below baseline values.

The term "variant," when used in the context of a polynucleotide sequence, may encompass a polynucleotide sequence related to a wild type gene. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. Of particular utility in the invention are variants of wild type gene products. Variants may result from at least one mutation in the nucleic acid sequence and may result in altered mRNAs or in polypeptides whose structure or function may or may not be altered. Any given natural or recombinant gene may have none, one, or many allelic forms. Common mutational changes that give rise to variants are generally ascribed to natural deletions, additions, or substitutions of nucleotides. Each of these types of changes may occur alone, or in combination with the others, one or more times in a given sequence.

The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs) or single base mutations in which the polynucleotide sequence varies by one base. The presence of SNPs may be indicative of, for example, a certain population with a propensity for a disease state, that is susceptibility versus resistance.

Derivative polynucleotides include nucleic acids subjected to chemical modification, for example, replacement of hydrogen by an alkyl, acyl, or amino group. Derivatives, e.g., derivative oligonucleotides, may comprise non-naturally-occurring portions, such as altered sugar moieties or inter-sugar linkages. Exemplary among these are phosphorothioate and other sulfur containing species which are known in the art. Derivative nucleic acids may also contain labels, including radionucleotides, enzymes, fluorescent agents, chemiluminescent agents, chromogenic agents, substrates, cofactors, inhibitors, magnetic particles, and the like.

A "derivative" polypeptide or peptide is one that is modified, for example, by glycosylation, pegylation, phosphorylation, sulfation, reduction/alkylation, acylation, chemical coupling, or mild formalin treatment. A derivative may also be modified to contain a detectable label, either directly or indirectly, including, but not limited to, a radioisotope, fluorescent, and enzyme label.

As used herein, the term "fragment or segment", as applied to a nucleic acid sequence, gene or polypeptide, will ordinarily be at least about 5 contiguous nucleic acid bases (for nucleic acid sequence or gene) or amino acids (for polypeptides), typically at least about 10 contiguous nucleic acid bases or amino acids, more typically at least about 20 contiguous nucleic acid bases or amino acids, usually at least about 30 contiguous nucleic acid bases or amino acids, preferably at least about 40 contiguous nucleic acid bases or amino acids, more preferably at least about 50 contiguous nucleic acid bases or amino acids, and even more preferably at least about 60 to 80 or more contiguous nucleic acid bases or amino acids in length. "Overlapping fragments" as used herein, refer to contiguous nucleic acid or peptide fragments which begin at the amino terminal end of a nucleic acid or protein and end at the carboxy terminal end of the nucleic acid or protein. Each nucleic acid or peptide fragment has at least about one contiguous nucleic acid or amino acid position in common with the next nucleic acid or peptide fragment, more preferably at least about three contiguous nucleic acid bases or amino acid positions in common, most preferably at least about ten contiguous nucleic acid bases amino acid positions in common.

The terms "biomolecule" or "markers" are used interchangeably herein and refer to DNA, RNA (including mRNA, rRNA, tRNA and tmRNA), nucleotides, nucleosides, analogs, polynucleotides, peptides and any combinations thereof.

"Expression/amount" of a gene, biomolecule, or biomarker in a first sample is at a level "greater than" the level in a second sample if the expression level/amount of the gene or biomarker in the first sample is at least about 1 time, 1.2 times, 1.5 times, 1.75 times, 2 times, 3 times , 4 times, 5 times, 6 times, 7 times, 8 times, 9 times, 10 times, 20 times, 30 times, the expression level/amount of the gene or biomarker in the second sample or a normal sample. Expression levels/amounts can be determined based on any suitable criterion known in the art, including but not limited to mRNA, cDNA, proteins, protein fragments and/or gene copy. Expression levels/amounts can be determined qualitatively and/or quantitatively.

The terms "detecting", "detect", "identifying", "quantifying" includes assaying, quantitating, imaging or otherwise establishing the presence or absence of the transcriptomic biomarker, or combinations of biomolecules comprising the biomarker, and the like, or assaying for, imaging, ascertaining, establishing, or otherwise determining the prognosis and/or diagnosis of renal diseases, disorders or conditions.

"Patient" or "subject" refers to mammals and includes human and veterinary subjects.

As used herein "a patient in need thereof" refers to any patient that is affected with a disorder characterized by proteinuria. In one aspect of the invention "a patient in need thereof refers to any patient that may have, or is at risk of having a disorder characterized by proteinuria.

As used herein, the term "test substance" or "candidate therapeutic agent" or "agent" are used interchangeably herein, and the terms are meant to encompass any molecule, chemical entity, composition, drug, therapeutic agent, chemotherapeutic agent, or biological agent capable of preventing, ameliorating, or treating a disease or other medical condition. The term includes small molecule compounds, antisense reagents, siRNA reagents, antibodies, enzymes, peptides organic or inorganic molecules, natural or synthetic compounds and the like. A test substance or agent can be assayed in accordance with the methods of the invention at any stage during clinical trials, during pre-trial testing, or following FDA-approval.

As used herein the phrase "diagnostic" means identifying the presence or nature of a pathologic condition. Diagnostic methods differ in their sensitivity and specificity. The "sensitivity" of a diagnostic assay is the percentage of diseased individuals who test positive (percent of "truce positives"). Diseased individuals not detected by the assay are "false negatives." Subjects who are not diseased and who test negative in the assay are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

As used herein the phrase "diagnosing" refers to classifying a disease or a symptom, determining a severity of the disease, monitoring disease progression, forecasting an outcome of a disease and/or prospects of recovery. The term "detecting" may also optionally encompass any of the above. Diagnosis of a disease according to the present invention can be effected by determining a level of a polynucleotide or a polypeptide of the present invention in a biological sample obtained from the subject, wherein the level determined can be correlated with predisposition to, or presence or absence of the disease. It should be noted that a "biological sample obtained from the subject" may also optionally comprise a sample that has not been physically removed from the subject, as described in greater detail below.

As defined herein, a therapeutically effective amount of a compound (i.e., an effective dosage) means an amount sufficient to produce a therapeutically (e.g., clinically) desirable result. The compositions can be administered one from one or more times per day to one or more times per week; including once every other day. The skilled artisan will appreciate that certain factors can influence the dosage and timing required to effectively treat a subject, including but not limited to the severity of the disease or disorder, previous treatments, the general health and/or age of the subject, and other diseases present. Moreover, treatment of a subject with a therapeutically effective amount of the compounds of the invention can include a single treatment or a series of treatments.

The term "sample" is meant to be interpreted in its broadest sense. A "sample" refers to a biological sample, such as, for example; one or more cells, tissues, or fluids (including, without limitation, plasma, serum, whole blood, cerebrospinal fluid, lymph, tears, urine, saliva, milk, pus, and tissue exudates and secretions) isolated from an individual or from cell culture constituents, as well as samples obtained from, for example, a laboratory procedure. A biological sample may comprise chromosomes isolated from cells (e.g., a spread of metaphase chromosomes), organelles or membranes isolated from cells, whole cells or tissues, nucleic acid such as genomic DNA in solution or bound to a solid support such as for Southern analysis, RNA in solution or bound to a solid support such as for Northern analysis, cDNA in solution or bound to a solid support, oligonucleotides in solution or bound to a solid support, polypeptides or peptides in solution or bound to a solid support, a tissue, a tissue print and the like.

Numerous well known tissue or fluid collection methods can be utilized to collect the biological sample from the subject in order to determine the level of DNA, RNA and/or polypeptide of the variant of interest in the subject. Examples include, but are not limited to, fine needle biopsy, needle biopsy, core needle biopsy and surgical biopsy (e.g., brain biopsy), and lavage. Regardless of the procedure employed, once a biopsy/sample is obtained the level of the variant can be determined and a diagnosis can thus be made.

The term "urokinase receptor molecule", "uPAR" is meant to include, soluble, membrane bound, variants, fragments, all family members, isoforms, precursors, mutants, alleles, fragments, species, sense and antisense polynucleotide strands, etc.

The term "neutralizing" when referring to an targeted binding agent such as an antibody relates to the ability of an antibody to eliminate, or significantly reduce, the activity of a target antigen. Accordingly, a "neutralizing" anti-uPAR antibody of the invention is capable of eliminating or significantly reducing the activity of uPAR. A neutralizing uPAR antibody may, for example, act by blocking the binding of uPA to its receptor uPAR. By blocking this binding, the uPA mediated plasminogen activation is significantly, or completely, eliminated.

"Active" or "activity" in regard to a uPAR polypeptide refers to a portion of an uPAR polypeptide that has a biological or an immunological activity of a native uPAR polypeptide. "Biological" when used herein refers to a biological function that results from the activity of the native uPAR polypeptide.

### Compositions

Proteinuria can be primarily caused by alterations of structural proteins involved in the cellular mechanism of filtration. The pathophysiological causes of proteinuria can be divided in the following major groups: (1) genetically determined disturbances of the structures which form the "glomerular filtration unit" like the glomerular basement membrane, the podocytes, or the slit diaphragm, (2) inflammatory processes, either directly caused by autoimmune processes or indirectly induced by microbes, (3) damage of the glomeruli caused by agents, or (4) as the final result of progressive tubulointerstitial injury finally resulting in the loss of function of the entire nephron.

Urokinase receptor (uPAR) signaling in podocytes has been recently shown to cause glomerular disease. In the examples which follow, in a mouse model of renal transplantation and in sera from patients that the soluble form of the urokinase receptor (suPAR) can be deposited in the kidney and cause proteinuric renal disease. Like endogenous podocyte uPAR, suPAR activates αᵥβ₃ integrin in an outside in dependent fashion. These studies identify the first circulating glomerular disease factor. Removal or blockade of this protein is a promising outlook for native organ maintenance as well as transplant survival.

uPAR is a glycosylphosphatidylinositol (GPI) -anchored protein with three extracellular domains. Cleavage of the GPI anchor generates suPAR. suPAR has been found elevated in sera of patients with HIV, rheumatic or neurological diseases, hematologic malignancies and epithelial tumors. The results herein, *inter alia,* show that the soluble form of uPAR also plays a role in proteinuric kidney disease similar to its membrane-anchored form.

uPAR is induced and activated in podocytes in response to proteinuric stimuli, such as LPS or PAN, leading to increased podocyte motility in vitro and FP effacement and proteinuria *in vivo.* Lipid raft-associated uPAR forms a complex with β3-integrin, thereby causing the activation of β3-integrin. This in turn promotes Cdc42 and Rac1 signaling, thereby causing podocyte FP effacement and proteinuria. Proteinuria caused by uPAR-β3-integrin signaling can be prevented and reduced by cycloRGDfV,42 a selective inhibitor of αᵥβ₃-integrin. The activation of Rac1 signaling in podocytes also contributes to proteinuria in human immunodeficiency virus-associated nephropathy and RhoGDIalpha knockout mice. Rac1-induced proteinuria can be blocked by the inhibition of Rac1 or blocking its downstream target aldosterone. Thus, the activation of promigratory Cdc42 and Rac1 signaling in podocytes is a widespread cause of FP effacement and proteinuria.

Phosphorylation of synaptopodin by PKA or CaMKII promotes 14-3-3 binding, which protects synaptopodin against CatL-mediated cleavage, thereby stabilizing synaptopodin steady-state levels. Synaptopodin suppresses IRSp53:Mena-mediated filopodia by blocking the binding of Cdc42 and Mena to IRSp53 and induces stress fibers by competitive blocking the Smurf-1-mediated ubiquitination of RhoA. Synaptopodin also prevents the CatL-mediated degradation of dynamin. Synaptopodin stabilizes the kidney filter by blocking the reorganization of the podocyte actin cytoskeleton into a migratory phenotype. Dephosphorylation of synaptopodin by calcineurin abrogates the interaction with 14-3-3. This renders the CatL cleavage sites of synaptopodin accessible and promotes the degradation of synaptopodin. LPS or various other proximal signals induce the expression of B7-1 and CatL in podocytes, which cause proteinuria through the increased degradation of synaptopodin and dynamin. In parallel, LPS or other proximal signals can also activate Cdc42 and Rac1 though uPAR:b3 integrin signaling, through the loss of synaptopodin-mediated inhibition of Cdc42 signaling or through Nef:Src-mediated activation of Rac1. As a consequence, the podocyte actin cytoskeleton shifts from a stationary to a motile phenotype, thereby causing foot process effacement and proteinuria. CsA and E64 safeguard against proteinuria by stabilizing synaptopodin and dynamin steady-state protein levels in podocytes, FP(4)-Mito by blocking Cdcd42:IRSp53:Mena signaling, cycloRGDfV by blocking uPAR:b3 integrin signaling, NSC23766 by blocking Rac1 and epleronone by blocking aldosterone signaling.

uPAR is activated in podocytes in response to proteinuric stimuli such as LPS or PAN, leading to increased podocyte motility *in vitro* as well as FP effacement and proteinuria *in vivo* and uPAR deficient mice are protected LPS-induced proteinuria. Activated uPAR associates with β3 integrin in lipid rafts thereby causing the activation of β3 integrin. Activated β3 integrin in turn induction of pro-migratory Cdc42 and Rac1 signaling, thereby causing podocyte FP effacement and proteinuria. uPAR:β3 integrin signaling-initiated proteinuria can be blocked by cycloRGDfV, a selective inhibitor of αᵥβ₃ integrin. Interestingly, the activation of Rac1 signaling in podocytes also contributes to proteinuria in HIV-associated nephropathy and RhoGDI knockout mice. Rac1-induced proteinuria can be blocked by inhibition of Rac1 or blocking its downstream target aldosterone. Thus, the activation of promigratory RhoGTPases Cdc42 and Rac1 in podocytes is a widespread cause of proteinuria.

Thus, in a preferred embodiment, a composition modulates expression and/or activity of membrane and/or soluble urokinase receptor molecules. Preferably, the agent modulates soluble urokinase receptor molecules. The agent can be any agent that modulates expression of urokinase receptor molecules or the activity of urokinase receptor molecules, such as for example, antisense oligonucleotides, antibodies, small molecules, and the like.

In a preferred embodiment, the agent modulates or inhibits urokinase receptor molecules expression, function and/or activity by about 5% as compared to a normal control, preferably by about 10%, preferably by about 50%, preferably by about 80%, 90%, 100%. Modulation of the, for example, soluble urokinase receptor molecules expression or amounts results in for example, a decrease in αvβ3 integrin activation and treatment of renal diseases such as proteinuria.

In another preferred embodiment an agent inhibits or blocks activated uPAR-β3-integrin signaling and podocyte FP hypermotility. This would be an example of a uPAR activity.

In another preferred embodiment, the agent modulates the degradation and/or rate of degradation of uPAR molecules as compared to normal controls by about 5%, preferably by about 50%, preferably by about 80%, 90%, 100%.

In another preferred embodiment, agents which modulate uPAR activity and/or expression comprise oligonucleotides, polynucleotides, peptides, polypeptides, antibodies, aptamers, small molecules, organic molecules, inorganic molecules or combinations thereof.

In another preferred embodiment, the composition comprises one or more agents which modulate uPAR expression, activity, and/or function *in vivo.* For example, one agent directly inhibits urokinase receptor molecules activity. In another example, an agent directly inhibits binding of uPAR to its ligand. In another preferred embodiment, a mimetic of a uPAR ligand binds to uPAR. In another preferred embodiment, a composition comprises an agent which directly targets urokinase receptor molecules, by, for example, binding to it, such as, an antibody, an antisense oligonucleotide which inhibits urokinase receptor molecules expression, a second agent which targets another molecule in the urokinase receptor molecules synthesis pathway, or molecules in pathways which are associated urokinase receptor molecules, such as for example, GTPase etc.

In another preferred embodiment, a method of treating a disease or disorder associated with pathological urokinase receptor molecules expression and/or activity comprises administering to a patient in need thereof, an effective amount of an agent which modulates urokinase receptor molecules activity, function and/or expression *in vivo* for treating the disorders. For example a podocyte disease or disorder such as proteinuria.

For example, the agent can be a vector expressing mutant uPAR molecules, an agent which targets uPAR nucleic acids which decrease *in vivo* production of uPAR.

In another preferred embodiment, a combination of agents which modulate uPAR expression, function and/or activity and/or modulate uPAR degradation are administered to a patient, for example, in the treatment of a disease or disorder characterized by proteinuria and/or podocyte diseases or disorders.

In a preferred embodiment, a disease or disorder characterized by proteinuria comprising: glomerular diseases, membranous glomerulonephritis, focal segmental glomerulonephritis, minimal change disease, nephrotic syndromes, pre-eclampsia, eclampsia, kidney lesions, collagen vascular diseases, stress, strenuous exercise, benign orthostatic (postural) proteinuria, focal segmental glomerulosclerosis (FSGS), IgA nephropathy, IgM nephropathy, membranoproliferative glomerulonephritis, membranous nephropathy, sarcoidosis, Alport's syndrome, diabetes mellitus, kidney damage due to drugs, Fabry's disease, infections, aminoaciduria, Fanconi syndrome, hypertensive nephrosclerosis, interstitial nephritis, Sickle cell disease, hemoglobinuria, multiple myeloma, myoglobinuria, cancer, Wegener's Granulomatosis or Glycogen Storage Disease Type 1.

In another preferred embodiment, modulation of uPAR expression, function, activity, etc, is modulated by an agent in the treatment of podocyte-related disorders or diseases. For the purposes of this invention, the terms "podocyte disease(s)" and "podocyte disorder(s)" are interchangeable and mean any disease, disorder, syndrome, anomaly, pathology, or abnormal condition of the podocytes or of the structure or function of their constituent parts.

In another preferred embodiment, a method of treating a podocyte disease or disorder associated with pathological urokinase receptor molecules expression and/or activity comprises administering to a patient in need thereof, an effective amount of an agent which modulates urokinase receptor molecules activity, function and/or expression *in vivo* for treating the podocyte diseases or disorders.

Such disorders or diseases include but are not limited to loss of podocytes (podocytopenia), podocyte mutation, an increase in foot process width, or a decrease in slit diaphragm length. In one aspect, the podocyte-related disease or disorder can be effacement or a diminution of podocyte density. In one aspect, the diminution of podocyte density could be due to a decrease in a podocyte number, for example, due to apoptosis, detachment, lack of proliferation, DNA damage or hypertrophy.

In one embodiment, the podocyte-related disease or disorder can be due to a podocyte injury. In one aspect, the podocyte injury can be due to mechanical stress such as high blood pressure, hypertension, or ischemia, lack of oxygen supply, a toxic substance, an endocrinologic disorder, an infection, a contrast agent, a mechanical trauma, a cytotoxic agent (cis-platinum, adriamycin, puromycin), calcineurin inhibitors, an inflammation (e.g., due to an infection, a trauma, anoxia, obstruction, or ischemia), radiation, an infection (e.g., bacterial, fungal, or viral), a dysfunction of the immune system (e.g., an autoimmune disease, a systemic disease, or IgA nephropathy), a genetic disorder, a medication (e.g., anti-bacterial agent, anti-viral agent, antifungal agent, immunosuppressive agent, anti-inflammatory agent, analgesic or anticancer agent), an organ failure, an organ transplantation, or uropathy. In one aspect, ischemia can be sickle-cell anemia, thrombosis, transplantation, obstruction, shock or blood loss. In on aspect, the genetic disorders may include congenital nephritic syndrome of the Finnish type, the fetal membranous nephropathy or mutations in podocyte-specific proteins, such as α-actin-4, podocin and TRPC6.

In one aspect, the podocyte-related disease or disorder can be an abnormal expression or function of slit diaphragm proteins such as podocin, nephrin, CD2AP, cell membrane proteins such as TRPC6, and proteins involved in organization of the cytoskeleton such as synaptopodin, actin binding proteins, lamb-families and collagens. In another aspect, the podocyte-related disease or disorder can be related to a disturbance of the GBM, to a disturbance of the mesangial cell function, and to deposition of antigen-antibody complexes and anti-podocyte antibodies. In another aspect, the podocyte-related disease or disorder can be tubular atrophy.

In a preferred embodiment, the podocyte-related disease or disorder comprises proteinuria, such as microalbumiuria or macroalbumiuria. Thus, in some preferred embodiments, one or more agents which modulate uPAR expression, function, activity, degradation, rate of degradation and/or inhibiting expression or activity of urokinase receptor molecules can be combined with one or more other chemotherapeutic compounds which are used to treat any of the podocyte diseases or disorders.

A wide variety of agents can be used to target uPARs, especially urokinase receptor molecules. These agents may be designed to target uPARs by having an *in vivo* activity which reduces the expression and/or activity of urokinase receptor molecules.

The agents may regulate urokinase receptor molecules based on the cDNA or regulatory regions of urokinase receptor molecules. For example, DNA-based agents, such as antisense inhibitors and ribozymes, can be utilized to target both the introns and exons of the uPAR genes as well as at the RNA level.

Alternatively, the agents may target urokinase receptor molecules based on the amino acid sequences including the propieces and/or three-dimensional protein structures of urokinase receptor molecules. Protein-based agents, such as human antibody, non-human monoclonal antibody and humanized antibody, can be used to specifically target different epitopes on urokinase receptor molecules. Peptides or peptidomimetics can serve as high affinity inhibitors to specifically bind to the active site of a particular uPAR, thereby inhibiting the *in vivo* activity of the uPAR. Small molecules may also be employed to target uPAR, especially those having high selectivity toward urokinase receptor molecules.

In addition to targeting urokinase receptor molecules, agents may also be used which competitively inhibit urokinase receptor molecules by competing with the natural ligands of urokinase receptor.

In another embodiment, one of the agents can be a are protease inhibitor, specific for urokinase receptor molecules.

*Antibodies*: In other embodiments of the invention described herein relate to targeted binding agents that bind uPAR and affect uPAR function. Examples include, monoclonal antibodies that bind uPAR and affect uPAR function. Other embodiments relate to anti-uPAR antibodies with high binding affinity for uPAR, the ability to neutralize uPAR *in vitro* and *in vivo*, and the ability to inhibit uPAR expression and/or function.

In another preferred embodiment, the invention relates to fully human anti-uPAR antibodies with desirable properties from a therapeutic perspective, including high binding affinity for uPAR, the ability to neutralize uPAR *in vitro* and *in vivo.*

In one embodiment, the invention includes antibodies that bind to uPAR with very high affinities (Kd). For example a human, rabbit, mouse, chimeric or humanized antibody that is capable of binding uPAR with a Kd less than, but not limited to, 10⁻⁵, 10⁻⁶, 10⁻⁷, 10⁻⁸, 10⁻⁹, 10⁻¹⁰ or 10⁻¹¹ M, or any range or value therein. Affinity and/or avidity measurements can be measured by KinExA™ and/or BIACORE™.

One embodiment of the invention includes isolated antibodies, or fragments of those antibodies, that bind to uPAR. As known in the art, the antibodies can be, for example, polyclonal, oligoclonal, monoclonal, chimeric, humanized, and/or fully human antibodies. Embodiments of the invention described herein also provide cells for producing these antibodies.

It will be appreciated that embodiments of the invention are not limited to any particular form of an antibody or method of generation or production. For example, the anti-uPAR antibody of the invention may be a full-length antibody (e.g., having an intact human Fc region) or an antibody fragment (e.g., a Fab, Fab', F(ab').sub.2, Fv or Dab (Dabs are the smallest functional binding units of human antibodies). In addition, the antibody may be manufactured from a hybridoma that secretes the antibody, or from a recombinantly produced cell that has been transformed or transfected with a gene or genes encoding the antibody.

Other embodiments of the invention include isolated nucleic acid molecules encoding any of the targeted binding agents, antibodies or fragments thereof as described herein, vectors having isolated nucleic acid molecules encoding anti-uPAR antibodies or a host cell transformed with any of such nucleic acid molecules. In addition, one embodiment of the invention is a method of producing an anti-uPAR antibody of the invention by culturing host cells under conditions wherein a nucleic acid molecule is expressed to produce the antibody followed by recovering the antibody. It should be realized that embodiments of the invention also include any nucleic acid molecule which encodes an antibody or fragment of an antibody of the invention including nucleic acid sequences optimized for increasing yields of antibodies or fragments thereof when transfected into host cells for antibody production.

A further embodiment includes a method of producing high affinity antibodies to uPAR by immunizing a mammal with human uPAR, or a fragment thereof, and one or more orthologous sequences or fragments thereof.

Another embodiment of the invention includes a method of diagnosing diseases or conditions in which an antibody prepared as described herein is utilized to detect the level of uPAR in a patient sample. In one embodiment, the patient sample is blood or blood serum. In further embodiments, methods for the identification of risk factors, diagnosis of disease, and staging of disease is presented which involves the identification of the overexpression of uPAR using anti-uPAR antibodies.
1. Another embodiment of the invention includes a method for diagnosing a condition associated with the expression of uPAR in a cell by contacting the serum or a cell with a targeted binding agent or an anti-uPAR antibody of the invention, and thereafter detecting the presence of uPAR. Preferred conditions include kidney disease comprises: podocyte diseases or disorders, proteinuria, glomerular diseases, membranous glomerulonephritis, focal segmental glomerulonephritis, minimal change disease, nephrotic syndromes, pre-eclampsia, eclampsia, kidney lesions, collagen vascular diseases, stress, strenuous exercise, benign orthostatic (postural) proteinuria, focal segmental glomerulosclerosis (FSGS), IgA nephropathy, IgM nephropathy, membranoproliferative glomerulonephritis, membranous nephropathy, sarcoidosis, Alport's syndrome, diabetes mellitus, kidney damage due to drugs, Fabry's disease, infections, aminoaciduria, Fanconi syndrome, hypertensive nephrosclerosis, interstitial nephritis, Sickle cell disease, hemoglobinuria, multiple myeloma, myoglobinuria, diabetic nephropathy (DN), lupus nephritis, Wegener's Granulomatosis or Glycogen Storage Disease Type 1.

In another embodiment, the invention includes an assay kit for detecting uPAR in mammalian tissues, cells, or body fluids to screen for uPAR-related diseases. The kit includes a targeted binding agent or an antibody of the invention that binds to uPAR and a means for indicating the reaction of the antibody with uPAR, if present. In one embodiment, the antibody is a monoclonal antibody. In another embodiment, the antibody that binds uPAR is labeled. In still another embodiment the antibody is an unlabeled primary antibody and the kit further includes a means for detecting the primary antibody. In one embodiment, the means for detecting includes a labeled second antibody that is an anti-immunoglobulin. The antibody may be labeled with a marker selected from the group consisting of a fluorochrome, an enzyme, a radionuclide and a radiopaque material.

Other embodiments of the invention include pharmaceutical compositions having an effective amount of a targeted binding agent or an anti-uPAR antibody of the invention in admixture with a pharmaceutically acceptable carrier or diluent. In yet other embodiments, the targeted binding agent or anti-uPAR antibody of the invention, or a fragment thereof, is conjugated to a therapeutic agent. The therapeutic agent can be, for example, a toxin or a radioisotope.
1. Yet another embodiment includes methods for treating diseases or conditions associated with the expression of uPAR in a patient, by administering to the patient an effective amount of a targeted binding agent or an anti-uPAR antibody of the invention. The targeted binding agent or anti-uPAR antibody of the invention can be administered alone, or can be administered in combination with additional antibodies or chemotherapeutic drug or radiation therapy. For example, a monoclonal, oligoclonal or polyclonal mixture of uPAR antibodies can be administered in combination with a drug shown to inhibit a disease state or symptoms associated therewith. The method can be performed *in vivo* and the patient is preferably a human patient. In a preferred embodiment, the method concerns the treatment of kidney disease comprises: podocyte diseases or disorders, proteinuria, glomerular diseases, membranous glomerulonephritis, focal segmental glomerulonephritis, minimal change disease, nephrotic syndromes, pre-eclampsia, eclampsia, kidney lesions, collagen vascular diseases, stress, strenuous exercise, benign orthostatic (postural) proteinuria, focal segmental glomerulosclerosis (FSGS), IgA nephropathy, IgM nephropathy, membranoproliferative glomerulonephritis, membranous nephropathy, sarcoidosis, Alport's syndrome, diabetes mellitus, kidney damage due to drugs, Fabry's disease, infections, aminoaciduria, Fanconi syndrome, hypertensive nephrosclerosis, interstitial nephritis, Sickle cell disease, hemoglobinuria, multiple myeloma, myoglobinuria, diabetic nephropathy (DN), lupus nephritis, Wegener's Granulomatosis or Glycogen Storage Disease Type 1.

In some embodiments, the targeted binding agent or anti-uPAR antibody of the invention is administered to a patient, followed by administration of a clearing agent to remove excess circulating antibody from the blood.

*Nucleic Acid-based Agents*: Nucleic acid-based agents such as antisense molecules and ribozymes can be utilized to target both the introns and exons of the uPAR genes as well as at the RNA level to inhibit gene expression thereof, thereby inhibiting the activity of the targeted uPAR. Further, triple helix molecules may also be utilized in inhibiting the uPAR gene activity. Such molecules may be designed to reduce or inhibit either the wild type uPAR gene, or if appropriate, the mutant uPAR gene activity. Techniques for the production and use of such molecules are well known to those of skill in the art, and are succinctly described below.

In another preferred embodiment, uPAR genes are modulated by targeting nucleic acid sequences involved in the expression and/or activity of uPAR molecules. For example, regulatory regions would be a target to decrease the expression of uPAR.

Antisense RNA and DNA molecules act to directly block the translation of mRNA by hybridizing to targeted mRNA and preventing protein translation. Antisense approaches involve the design of oligonucleotides that are complementary to a target gene mRNA. The antisense oligonucleotides will bind to the complementary target gene mRNA transcripts and prevent translation. Absolute complementarity, although preferred, is not required.

A sequence "complementary" to a portion of an RNA, as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the message, e.g., the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. Wagner (1994) Nature 372:333-335. For example, oligonucleotides complementary to either the 5'- or 3'-untranslated, non-coding regions of the human or mouse gene of urokinase receptor molecules could be used in an antisense approach to inhibit translation of endogenous urokinase receptor molecules mRNA.

In another preferred embodiment, the antisense approach can be used to target negative regulators of uPAR expression and/or function.

Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Whether designed to hybridize to the 5'-, 3'- or coding region of target gene mRNA, antisense nucleic acids are preferably at least six nucleotides in length, and are more preferably oligonucleotides ranging from 6 to about 50 nucleotides in length. In specific aspects the oligonucleotide is at least 10 nucleotides, preferably at least 17 nucleotides, more preferably at least 25 nucleotides and most preferably at least 50 nucleotides.

Alternatively, antisense molecules may be designed to target the translated region, i.e., the cDNA of the uPAR gene. For example, the antisense RNA molecules targeting the full coding sequence or a portion of the mature murine urokinase receptor molecules (Kirschke et al. (2000) Euro. J. Cancer 36:787-795) may be utilized to inhibit expression of urokinase receptor molecules and thus reduce the activity of its enzymatic activity. In addition, a full length or partial urokinase receptor molecules cDNA can be subcloned into a pcDNA-3 expression vector in reversed orientation and such a construct can be transfected into cells to produce antisense polyRNA to block endogenous transcripts of a uPAR, such as urokinase receptor molecules, and thus inhibit uPAR's expression.

*In vitro* studies may be performed to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides, or agents facilitating transport across the cell membrane (See, e.g., Letsinger (1989) Proc. Natl. Acad. Sci. U.S.A. 86:6553-6556) or the blood-brain barrier, hybridization-triggered cleavage agents. See, e.g., Krol (1988) Bio Techniques 6:958-976 or intercalating agents. See, e.g., Zon (1988) Pharm. Reps. 5:539-549. The oligonucleotide may be conjugated to another molecule, e.g., a peptide, hybridization triggered cross-linking agent, transport agent, hybridization-triggered cleavage agent, etc.

The antisense oligonucleotide may comprise at least one modified base moiety which is selected from the group consisting of, but not being limited to, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomet- hyluracil, dihydrouracil, β-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, β-D-mannosylqueosine, 5'-methoxycarboxymethyluracil, 5-methoxyuracil, 2-methylthio-N6-isopenten- yladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group consisting of, but not being limited to arabinose, 2-fluoroarabinose, xylulose, and hexose.

In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

Ribozyme molecules designed to catalytically cleave target gene mRNA transcripts can also be used to prevent translation of target gene mRNA and, therefore, expression of target gene product. See, e.g. Sarver et al. (1990) Science 247:1222-1225.

Ribozymes are enzymatic RNA molecules capable of catalyzing the specific cleavage of RNA. The mechanism of ribozyme action involves sequence specific hybridization of the ribozyme molecule to complementary target RNA, followed by an endonucleolytic cleavage event. The composition of ribozyme molecules should include one or more sequences complementary to the target gene mRNA, and should include the well known catalytic sequence responsible for mRNA cleavage.

While ribozymes that cleave mRNA at site specific recognition sequences can be used to destroy target gene mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art.

Endogenous uPAR gene expression can also be reduced by inactivating or "knocking out" the targeted uPAR gene or its promoter using targeted homologous recombination. Smithies et al. (1985) Nature 317:230-234; Thomas and Capecchi, (1987) Cell 51:503-512; and Thompson et al. (1989) Cell 5:313-321.

Alternatively, endogenous uPAR gene expression can be reduced by targeting deoxyribonucleotide sequences complementary to the regulatory region of the uPAR gene (i.e., the target gene promoter and/or enhancers) to form triple helical structures that prevent transcription of the target gene in target cells in the body. See generally, Helene (1991) Anticancer Drug Des. 6:569-584; Helene et al. (1992) Ann. N.Y. Acad. Sci. 660:27-36; and Maher (1992) Bioassays 14:807-815.

Nucleic acid molecules to be used in triplex helix formation for the inhibition of transcription should be single stranded and composed of deoxynucleotides. The base composition of these oligonucleotides must be designed to promote triple helix formation via Hoögsteen base pairing rules, which generally require sizeable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, contain a stretch of G residues. These molecules will form a triple helix with a DNA duplex that is rich in GC pairs, in which the majority of the purine residues are located on a single strand of the targeted duplex, resulting in GGC triplets across the three strands in the triplex.

### Biomarkers

In a preferred embodiment, a biomarker for the diagnosis of a disease or disorder characterized by proteinuria and/or identification of individuals at risk of developing a disease or disorder characterized by proteinuria comprising: uPAR, dynamin, synaptopodin, variants, mutants or fragments thereof.

The biomarkers can be increased or decreased in expression relative to each other. The panel of biomarker expression profiles are compared to normal controls. In other instances, the intra-cellular localization changes with the progression of disease.

In another preferred embodiment, the identification of an individual at risk of developing disease or disorder characterized by proteinuria detects at least one biomarker or fragments thereof.

In another preferred embodiment, the progression of disease or disorder characterized by proteinuria is correlated to an increase in uPAR.

### Candidate Therapeutic Agents:

In a preferred embodiment, methods (also referred to herein as "screening assays") are provided for identifying modulators, i.e., candidate or test compounds or agents (e.g., proteins, peptides, peptidomimetics, peptoids, small molecules, analogues or other drugs) which modulate uPAR expression, function degradation and/or act directly on urokinase receptor molecules activity or expression or synthesis pathways thereof. Compounds thus identified can be used to modulate the activity of target gene products, prolong the half-life of a protein or peptide, regulate cell division, etc, in a therapeutic protocol, to elaborate the biological function of the target gene product, or to identify compounds that disrupt normal target gene interactions.

In another preferred embodiment, a high-throughput screening assay (HTS) screening assay is used to screen a diverse library of member compounds. The "compounds" or "candidate therapeutic agents" or "candidate agents" can be any organic, inorganic, small molecule, protein, antibody, aptamer, nucleic acid molecule, or synthetic compound.

In another preferred embodiment, the candidate agents modulate uPAR enzymes, precursors or molecules involved in the pathways. Preferably, the enzyme is urokinase receptor molecules. These enzymes can be involved in various biochemical pathways such as synthetic pathways, breakdown pathways, e.g. ubiquitin, enzymatic pathways, protein trafficking pathways, metabolic pathways, signal transduction pathways, and the like.

In another preferred embodiment, the high throughput assays identifies candidate agents that target and modulate the pathways involved in the pathological expression or activity of urokinase receptor molecules The candidate agents would be useful in developing and identifying novel agents for the treatment of podocyte diseases or disorders, such as, for example, proteinuria.

In one embodiment, the invention provides assays for screening candidate or test compounds which modulate the degradation, rate of degradation, activity, expression and/or function of uPAR molecules, including for example circulating uPAR.

In another embodiment, the invention provides assays for screening candidate or test compounds that bind to or modulate an activity of urokinase receptor molecules protein or polypeptide or a biologically active portion thereof, mutants or fragments, or fusion proteins thereof.

Candidate agents include numerous chemical classes, though typically they are organic compounds including small organic compounds, nucleic acids including oligonucleotides, and peptides. Small organic compounds suitably may have e.g. a molecular weight of more than about 40 or 50 yet less than about 2,500. Candidate agents may comprise functional chemical groups that interact with proteins and/or DNA.

The test compounds of the present invention can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, e.g., Zuckermann, R. N. et al. (1994) J. Med. Chem. 37:2678-85); spatially addressable parallel solid phase or solution phase libraries; synthetic library methods requiring deconvolution; the one-bead one-compound library method; and synthetic library methods using affinity chromatography selection. The biological library and peptoid library approaches are limited to peptide libraries, while the other four approaches are applicable to peptide, non-peptide oligomer or small molecule libraries of compounds (Lam (1997) Anticancer Drug Des. 12:145).

Examples of methods for the synthesis of molecular libraries can be found in the art, for example in: DeWitt et al. (1993) Proc. Natl. Acad Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and Gallop et al. (1994) J. Med. Chem. 37:1233.

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner, U.S. Pat. No. 5,223,409), spores (Ladner U.S. Pat. No. 5,223,409), plasmids (Cull et al. (1992) Proc Nat'l Acad Sci USA 89:1865-1869) or on phage (Scott and Smith (1990) Science 249:386-390; Devlin (1990) Science 249:404-406; Cwirla et al. (1990) Proc. Natl. Acad. Sci. 87:6378-6382; Felici (1991) J. Mol. Biol. 222:301-310; Ladner *supra.*)*.*

In another preferred embodiment, the candidate therapeutic agent comprises, proteins, peptides, organic molecules, inorganic molecules, nucleic acid molecules, and the like. These molecules can be natural, e.g. from plants, fungus, bacteria etc., or can be synthesized or synthetic.

A prototype compound may be believed to have therapeutic activity on the basis of any information available to the artisan. For example, a prototype compound may be believed to have therapeutic activity on the basis of information contained in the Physician's Desk Reference. In addition, by way of non-limiting example, a compound may be believed to have therapeutic activity on the basis of experience of a clinician, structure of the compound, structural activity relationship data, EC₅₀, assay data, IC₅₀ assay data, animal or clinical studies, or any other basis, or combination of such bases.

A therapeutically-active compound is a compound that has therapeutic activity, including for example, the ability of a compound to induce a specified response when administered to a subject or tested *in vitro.* Therapeutic activity includes treatment of a disease or condition, including both prophylactic and ameliorative treatment. Treatment of a disease or condition can include improvement of a disease or condition by any amount, including prevention, amelioration, and elimination of the disease or condition. Therapeutic activity may be conducted against any disease or condition, including in a preferred embodiment against any disease or disorder associated with proteinuria. In order to determine therapeutic activity any method by which therapeutic activity of a compound may be evaluated can be used. For example, both *in vivo* and *in vitro* methods can be used, including for example, clinical evaluation, EC₅₀, and IC₅₀ assays, and dose response curves.

Candidate compounds for use with an assay of the present invention or identified by assays of the present invention as useful pharmacological agents can be pharmacological agents already known in the art or variations thereof or can be compounds previously unknown to have any pharmacological activity. The candidate compounds can be naturally occurring or designed in the laboratory. Candidate compounds can comprise a single diastereomer, more than one diastereomer, or a single enantiomer, or more than one enantiomer.

Candidate compounds can be isolated, from microorganisms, animals or plants, for example, and can be produced recombinantly, or synthesized by chemical methods known in the art. If desired, candidate compounds of the present invention can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries. The other four approaches are applicable to polypeptide, non-peptide oligomer, or small molecule libraries of compounds and are preferred approaches in the present invention. See Lam, Anticancer Drug Des. 12: 145-167 (1997).

In an embodiment, the present invention provides a method of identifying a candidate compound as a suitable prodrug. A suitable prodrug includes any prodrug that may be identified by the methods of the present invention. Any method apparent to the artisan may be used to identify a candidate compound as a suitable prodrug.

In another aspect, the present invention provides methods of screening candidate compounds for suitability as therapeutic agents. Screening for suitability of therapeutic agents may include assessment of one, some or many criteria relating to the compound that may affect the ability of the compound as a therapeutic agent. Factors such as, for example, efficacy, safety, efficiency, retention, localization, tissue selectivity, degradation, or intracellular persistence may be considered. In an embodiment, a method of screening candidate compounds for suitability as therapeutic agents is provided, where the method comprises providing a candidate compound identified as a suitable prodrug, determining the therapeutic activity of the candidate compound, and determining the intracellular persistence of the candidate compound. Intracellular persistence can be measured by any technique apparent to the skilled artisan, such as for example by radioactive tracer, heavy isotope labeling, or LCMS.

In screening compounds for suitability as therapeutic agents, intracellular persistence of the candidate compound is evaluated. In a preferred embodiment, the agents are evaluated for their ability to modulate the intracellular pH may comprise, for example, evaluation of intracellular pH over a period of time in response to a candidate therapeutic agent. In a preferred embodiment, the intra-podocyte pH in the presence or absence of the candidate therapeutic compound in human tissue is determined. Any technique known to the art worker for determining intracellular pH may be used in the present invention. See, also, the experimental details in the examples section which follows.

A further aspect of the present invention relates to methods of inhibiting the activity of a condition or disease associated with proteinuria comprising the step of treating a sample or subject believed to have a disease or condition with a prodrug identified by a compound of the invention. Compositions of the invention act as identifiers for prodrugs that have therapeutic activity against a disease or condition. In a preferred aspect, compositions of the invention act as identifiers for drugs that show therapeutic activity against conditions including for example associated with proteinuria.

In one embodiment, a screening assay is a cell-based assay in which the activity of urokinase receptor molecules is measured against an increase or decrease of pH values in the cells. Determining the ability of the test compound to modulate the pH and determining urokinase receptor molecules activity, by various methods, including for example, fluorescence, protein assays, blots and the like. The cell, for example, can be of mammalian origin, e.g., human.

In another preferred embodiment, the screening assay is a high-throughput screening assay. The ability of a compound to modulate uPAR degradation, expression, function etc., and/or modulate urokinase receptor molecules expression and/or activity can be evaluated as described in detail in the Examples which follow.

In another preferred embodiment, soluble and/or membrane-bound forms of isolated proteins, mutants or biologically active portions thereof, can be used in the assays if desired. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, TRITON™ X-100, Tritons X-114, THESIT™, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

Cell-free assays can also be used and involve preparing a reaction mixture which includes urokinase receptor molecules, and the test compound under conditions and time periods to allow the measurement of the urokinase receptor molecules activity over time, etc, over a range of values and concentrations of test agents.

The enzymatic activity can be also be detected, e.g., using fluorescence energy transfer (FET) (see, for example, Lakowicz et al., U.S. Pat. No. 5,631,169; Stavrianopoulos, et al, U.S. Pat. No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. A FET binding event can be conveniently measured through standard fluorometric detection means well known in the art (e.g., using a fluorimeter).

In another embodiment, determining the ability of the enzyme (e.g. urokinase receptor molecules) to bind or "dock" to its binding site on a target molecule (uPAR) can be accomplished using real-time Biomolecular Interaction Analysis (BIA) (see, e.g., Sjolander, S. and Urbaniczky, C. (1991) Anal. Chem. 63:2338-2345 and Szabo et al. (1995) Curr. Opin. Struct. Biol. 5:699-705). "Surface plasmon resonance" or "BIA" detects biospecific interactions in real time, without labeling any of the interactants (e.g., BLAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target product or the test substance is anchored onto a solid phase. The target product/test compound complexes anchored on the solid phase can be detected at the end of the reaction. Preferably, the target product can be anchored onto a solid surface, and the test compound, (which is not anchored), can be labeled, either directly or indirectly, with detectable labels discussed herein.

Candidate agents may be obtained from a wide variety of sources including libraries of synthetic or natural compounds. For example, numerous means are available for random and directed synthesis of a wide variety of organic compounds and biomolecules, including expression of randomized oligonucleotides. Alternatively, libraries of natural compounds in the form of e.g. bacterial, fungal and animal extracts are available or readily produced.

*Chemical Libraries*: Developments in combinatorial chemistry allow the rapid and economical synthesis of hundreds to thousands of discrete compounds. These compounds are typically arrayed in moderate-sized libraries of small molecules designed for efficient screening. Combinatorial methods can be used to generate unbiased libraries suitable for the identification of novel compounds. In addition, smaller, less diverse libraries can be generated that are descended from a single parent compound with a previously determined biological activity. In either case, the lack of efficient screening systems to specifically target therapeutically relevant biological molecules produced by combinational chemistry such as inhibitors of important enzymes hampers the optimal use of these resources.

A combinatorial chemical library is a collection of diverse chemical compounds generated by either chemical synthesis or biological synthesis, by combining a number of chemical "building blocks," such as reagents. For example, a linear combinatorial chemical library, such as a polypeptide library, is formed by combining a set of chemical building blocks (amino acids) in a large number of combinations, and potentially in every possible way, for a given compound length (i.e., the number of amino acids in a polypeptide compound). Millions of chemical compounds can be synthesized through such combinatorial mixing of chemical building blocks.

A "library" may comprise from 2 to 50,000,000 diverse member compounds. Preferably, a library comprises at least 48 diverse compounds, preferably 96 or more diverse compounds, more preferably 384 or more diverse compounds, more preferably, 10,000 or more diverse compounds, preferably more than 100,000 diverse members and most preferably more than 1,000,000 diverse member compounds. By "diverse" it is meant that greater than 50% of the compounds in a library have chemical structures that are not identical to any other member of the library. Preferably, greater than 75% of the compounds in a library have chemical structures that are not identical to any other member of the collection, more preferably greater than 90% and most preferably greater than about 99%.

The preparation of combinatorial chemical libraries is well known to those of skill in the art. For reviews, see Thompson et al., Synthesis and application of small molecule libraries, Chem Rev 96:555-600, 1996; Kenan et al., Exploring molecular diversity with combinatorial shape libraries, Trends Biochem Sci 19:57-64, 1994; Janda, Tagged versus untagged libraries: methods for the generation and screening of combinatorial chemical libraries, Proc Natl Acad Sci USA. 91:10779-85, 1994; Lebl et al., One-bead-one-structure combinatorial libraries, Biopolymers 37:177-98, 1995; Eichler et al., Peptide, peptidomimetic, and organic synthetic combinatorial libraries, Med Res Rev. 15:481-96, 1995; Chabala, Solid-phase combinatorial chemistry and novel tagging methods for identifying leads, Curr Opin Biotechnol. 6:632-9, 1995; Dolle, Discovery of enzyme inhibitors through combinatorial chemistry, Mol Divers. 2:223-36, 1997; Fauchere et al., Peptide and nonpeptide lead discovery using robotically synthesized soluble libraries, Can J. Physiol Pharmacol. 75:683-9, 1997; Eichler et al., Generation and utilization of synthetic combinatorial libraries, Mol Med Today 1: 174-80, 1995; and Kay et al., Identification of enzyme inhibitors from phage-displayed combinatorial peptide libraries, Comb Chem High Throughput Screen 4:535-43, 2001.

Other chemistries for generating chemical diversity libraries can also be used. Such chemistries include, but are not limited to, peptoids (PCT Publication No. WO 91/19735); encoded peptides (PCT Publication WO 93/20242); random bio-oligomers (PCT Publication No. WO 92/00091); benzodiazepines (U.S. Pat. No. 5,288,514); diversomers, such as hydantoins, benzodiazepines and dipeptides (Hobbs, et al., Proc. Nat. Acad. Sci. USA, 90:6909-6913 (1993)); vinylogous polypeptides (Hagihara, et al., J. Amer. Chem. Soc. 114:6568 (1992)); nonpeptidal peptidomimetics with β-D-glucose scaffolding (Hirschmann, et al., J. Amber. Chem. Soc., 114:9217-9218 (1992)); analogous organic syntheses of small compound libraries (Chen, et al., J. Amer. Chem. Soc., 116:2661 (1994)); oligocarbamates (Cho, et al. Science, 261:1303 (1993)); and/or peptidyl phosphonates (Campbell, et al., J. Org. Chem. 59:658 (1994)); nucleic acid libraries (see, Ausubel, Berger and Sambrook, all *supra*); peptide nucleic acid libraries (see, e.g., U.S. Pat. No. 5,539,083); antibody libraries (see, e.g., Vaughn, et al., Nature Biotechnology, 14(3):309-314 (1996) and PCT/US96/10287); carbohydrate libraries (see, e.g., Liang, et al, Science, 274:1520-1522 (1996) and U.S. Pat. No. 5,593,853); small organic molecule libraries (see, e.g., benzodiazepines, Baum C&E News, January 18, page 33 (1993); isoprenoids (U.S. Pat. No. 5,569,588); thiazolidinones and metathiazanones (U.S. Pat. No. 5,549,974); pyrrolidines (U.S. Pat. Nos. 5,525,735 and 5,519,134); morpholino compounds (U.S. Pat. No. 5,506,337); benzodiazepines (U.S. Pat. No. 5,288,514); and the like.

Devices for the preparation of combinatorial libraries are commercially available (see, e.g., 357 MPS, 390 MPS, Advanced Chem. Tech, Louisville Ky., Symphony, Rainin, Woburn, Mass., 433A Applied Biosystems, Foster City, Calif., 9050 Plus, Millipore, Bedford, Mass.). In addition, numerous combinatorial libraries are themselves commercially available (see, e.g., ComGenex, Princeton, N.J., Asinex, Moscow, Ru, Tripos, Inc., St. Louis, Mo., ChemStar, Ltd., Moscow, RU, 3D Pharmaceuticals, Exton, Pa., Martek Bio sciences, Columbia, Md., etc.).

*Small Molecules*: Small molecule test compounds can initially be members of an organic or inorganic chemical library. As used herein, "small molecules" refers to small organic or inorganic molecules of molecular weight below about 3,000 Daltons. The small molecules can be natural products or members of a combinatorial chemistry library. A set of diverse molecules should be used to cover a variety of functions such as charge, aromaticity, hydrogen bonding, flexibility, size, length of side chain, hydrophobicity, and rigidity. Combinatorial techniques suitable for synthesizing small molecules are known in the art, e.g., as exemplified by Obrecht and Villalgordo, Solid-Supported Combinatorial and Parallel Synthesis of Small-Molecular-Weight Compound Libraries, Pergamon-Elsevier Science Limited (1998), and include those such as the "split and pool" or "parallel" synthesis techniques, solid-phase and solution-phase techniques, and encoding techniques (see, for example, Czarnik, Curry Opin. Chef. Bio., 1:60 (1997). In addition, a number of small molecule libraries are commercially available.

The whole procedure can be fully automated. For example, sampling of sample materials may be accomplished with a plurality of steps, which include withdrawing a sample from a sample container and delivering at least a portion of the withdrawn sample to test platform. Sampling may also include additional steps, particularly and preferably, sample preparation steps. In one approach, only one sample is withdrawn into the auto-sampler probe at a time and only one sample resides in the probe at one time. In other embodiments, multiple samples may be drawn into the auto-sampler probe separated by solvents. In still other embodiments, multiple probes may be used in parallel for auto sampling.

In the general case, sampling can be effected manually, in a semi-automatic manner or in an automatic manner. A sample can be withdrawn from a sample container manually, for example, with a pipette or with a syringe-type manual probe, and then manually delivered to a loading port or an injection port of a characterization system. In a semi-automatic protocol, some aspect of the protocol is effected automatically (e.g., delivery), but some other aspect requires manual intervention (e.g., withdrawal of samples from a process control line). Preferably, however, the sample(s) are withdrawn from a sample container and delivered to the characterization system, in a fully automated manner - for example, with an auto-sampler.

In one embodiment, auto-sampling may be done using a microprocessor controlling an automated system (e.g., a robot arm). Preferably, the microprocessor is user-programmable to accommodate libraries of samples having varying arrangements of samples (e.g., square arrays with "n-rows" by "n-columns," rectangular arrays with "n-rows" by "m-columns," round arrays, triangular arrays with "r-" by "r-" by "r-" equilateral sides, triangular arrays with "r-base" by "s-" by "s-" isosceles sides, etc., where n, m, r, and s are integers).

Automated sampling of sample materials optionally may be effected with an auto-sampler having a heated injection probe (tip). An example of one such auto sampler is disclosed in U.S. Pat. No. 6,175,409 B1 (incorporated by reference).

According to the present invention, one or more systems, methods or both are used to identify a plurality of sample materials. Though manual or semi-automated systems and methods are possible, preferably an automated system or method is employed. A variety of robotic or automatic systems are available for automatically or programmably providing predetermined motions for handling, contacting, dispensing, or otherwise manipulating materials in solid, fluid liquid or gas form according to a predetermined protocol. Such systems may be adapted or augmented to include a variety of hardware, software or both to assist the systems in determining mechanical properties of materials. Hardware and software for augmenting the robotic systems may include, but are not limited to, sensors, transducers, data acquisition and manipulation hardware, data acquisition and manipulation software and the like. Exemplary robotic systems are commercially available from CAVRO Scientific Instruments (e.g., Model NO. RSP9652) or BioDot (Microdrop Model 3000).

Generally, the automated system includes a suitable protocol design and execution software that can be programmed with information such as synthesis, composition, location information or other information related to a library of materials positioned with respect to a substrate. The protocol design and execution software is typically in communication with robot control software for controlling a robot or other automated apparatus or system. The protocol design and execution software is also in communication with data acquisition hardware/software for collecting data from response measuring hardware. Once the data is collected in the database, analytical software may be used to analyze the data, and more specifically, to determine properties of the candidate drugs, or the data may be analyzed manually.

*Data and Analysis:* The practice of the present invention may also employ conventional biology methods, software and systems. Computer software products of the invention typically include computer readable medium having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, for example Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001). See U.S. Pat. No. 6,420,108.

The present invention may also make use of various computer program products and software for a variety of purposes, such as probe design, management of data, analysis, and instrument operation. See, U.S. Pat. Nos. 5,593,839, 5,795,716, 5,733,729, 5,974,164, 6,066,454, 6,090,555, 6,185,561, 6,188,783, 6,223,127, 6,229,911 and 6,308,170.

Additionally, the present invention relates to embodiments that include methods for providing genetic information over networks such as the Internet.

### Administration of Compositions to Patients

The compositions or agents identified by the methods described herein may be administered to animals including human beings in any suitable formulation. For example, the compositions for modulating protein degradation may be formulated in pharmaceutically acceptable carriers or diluents such as physiological saline or a buffered salt solution. Suitable carriers and diluents can be selected on the basis of mode and route of administration and standard pharmaceutical practice. A description of exemplary pharmaceutically acceptable carriers and diluents, as well as pharmaceutical formulations, can be found in Remington's Pharmaceutical Sciences, a standard text in this field, and in USP/NF. Other substances may be added to the compositions to stabilize and/or preserve the compositions.

The compositions of the invention may be administered to animals by any conventional technique. The compositions may be administered directly to a target site by, for example, surgical delivery to an internal or external target site, or by catheter to a site accessible by a blood vessel. Other methods of delivery, e.g., liposomal delivery or diffusion from a device impregnated with the composition, are known in the art. The compositions may be administered in a single bolus, multiple injections, or by continuous infusion (e.g., intravenously). For parenteral administration, the compositions are preferably formulated in a sterilized pyrogen-free form.

The compounds can be administered with one or more therapies. The chemotherapeutic agents may be administered under a metronomic regimen. As used herein, "metronomic" therapy refers to the administration of continuous low-doses of a therapeutic agent.

Dosage, toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds that exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of a compound (i.e., an effective dosage) means an amount sufficient to produce a therapeutically (e.g., clinically) desirable result. The compositions can be administered one from one or more times per day to one or more times per week; including once every other day. The skilled artisan will appreciate that certain factors can influence the dosage and timing required to effectively treat a subject, including but not limited to the severity of the disease or disorder, previous treatments, the general health and/or age of the subject, and other diseases present. Moreover, treatment of a subject with a therapeutically effective amount of the compounds of the invention can include a single treatment or a series of treatments.

### Formulations

While it is possible for a composition to be administered alone, it is preferable to present it as a pharmaceutical formulation. The active ingredient may comprise, for topical administration, from 0.001% to 10% w/w, e.g., from 1% to 2% by weight of the formulation, although it may comprise as much as 10% w/w but preferably not in excess of 5% w/w and more preferably from 0.1% to 1% w/w of the formulation. The topical formulations of the present invention, comprise an active ingredient together with one or more acceptable carrier(s) therefor and optionally any other therapeutic ingredients(s). The carrier(s) must be "acceptable" in the sense of being compatible with the other ingredients of the formulation and not deleterious to the recipient thereof.

Formulations suitable for topical administration include liquid or semi-liquid preparations suitable for penetration through the skin to the site of where treatment is required, such as liniments, lotions, creams, ointments or pastes, and drops suitable for administration to the eye, ear, or nose. Drops according to the present invention may comprise sterile aqueous or oily solutions or suspensions and may be prepared by dissolving the active ingredient in a suitable aqueous solution of a bactericidal and/or fungicidal agent and/or any other suitable preservative, and preferably including a surface active agent. The resulting solution may then be clarified and sterilized by filtration and transferred to the container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

Lotions according to the present invention include those suitable for application to the skin or eye. An eye lotion may comprise a sterile aqueous solution optionally containing a bactericide and may be prepared by methods similar to those for the preparation of drops. Lotions or liniments for application to the skin may also include an agent to hasten drying and to cool the skin, such as an alcohol or acetone, and/or a moisturizer such as glycerol or an oil such as castor oil or arachis oil.

Creams, ointments or pastes according to the present invention are semi-solid formulations of the active ingredient for external application. They may be made by mixing the active ingredient in finely-divided or powdered form, alone or in solution or suspension in an aqueous or non-aqueous fluid, with the aid of suitable machinery, with a greasy or non-greasy basis. The basis may comprise hydrocarbons such as hard, soft or liquid paraffin, glycerol, beeswax, a metallic soap; a mucilage; an oil of natural origin such as almond, corn, arachis, castor or olive oil; wool fat or its derivatives, or a fatty acid such as stearic or oleic acid together with an alcohol such as propylene glycol or macrogels. The formulation may incorporate any suitable surface active agent such as an anionic, cationic or non-ionic surface active such as sorbitan esters or polyoxyethylene derivatives thereof. Suspending agents such as natural gums, cellulose derivatives or inorganic materials such as silicaceous silicas, and other ingredients such as lanolin, may also be included.

While various embodiments of the present invention have been described above, it should be understood that they have been presented by way of example only, and not limitation. Numerous changes to the disclosed embodiments can be made in accordance with the disclosure herein without departing from the spirit or scope of the invention. Thus, the breadth and scope of the present invention should not be limited by any of the above described embodiments.

All documents mentioned herein are incorporated herein by reference. All publications and patent documents cited in this application are incorporated by reference for all purposes to the same extent as if each individual publication or patent document were so individually denoted. By their citation of various references in this document, Applicants do not admit any particular reference is "prior art" to their invention. Embodiments of inventive compositions and methods are illustrated in the following examples.

### EXAMPLES

The following non-limiting Examples serve to illustrate selected embodiments of the invention. It will be appreciated that variations in proportions and alternatives in elements of the components shown will be apparent to those skilled in the art and are within the scope of embodiments of the present invention.

### Example 1: Soluble uP AR is a glomerular disease recurrence factor

### Materials and Methods:

*Antibodies:* active β3 integrin antibody (AP5), (GTI); uPAR (FL-290) (Santa Cruz
Biotechnology); synaptopodin mouse monoclonal antibody (G1), synaptopodin rabbit polyclonal (NT); Quantikine human uPAR immunoassay kit (R&D Systems); suPAR protein.

*Animals and treatments:* All animal studies were approved by the Subcommittee on Research Animal Care of the Massachusetts General Hospital, protocol number: 2004N000289/2. *Plaur*^{*-*/*-*} mice were obtained ,from University of Leuven, Belgium; 57BL/6 mice were purchased from the Jackson Laboratory. LPS mouse model were utilized as previously described (Reiser, J. et al, J. Clin Invest. 113, 1390-7 (2004). *In vivo* suPAR protein delivery was done with *Plaur* ^{-/-} female mice (*n* = 6) through tail vein at 1 mg/kg body weight. Controls *(n* = 6) received the same amount of BSA. Urine was collected before and 8 h, 24 h after suPAR or BSA injection. Urine protein concentration was determined by Braford assay (Sigma).

*Right kidney cross*-*transplantation:* Six 57BL/6 female mice and 6 *Plaur* ^{*-*/*-*} female mice in the same age and body weight ranges (7-8 weeks, 15-18 g) were randomly recruited into this study. The right kidney from *Plaur* ^{-/-} mice were transplanted into 57BL/6 mice at the right side and *vice versa.* Kidney transplantation was performed as previously described (Coffman, T. et al, J. Immunol. 151, 425-435 (1993); Han, W.R., Murray-Segal, L.J. and Mottram, P.L. Microsurgery. 19, 272-274 (1999)). After recovery from surgery, mice were randomly treated with LPS at 10 mg/kg body weight, i.p. or same amount of PBS for control. 24 h after LPS injection, mice were sacrificed, and kidney was taken out for further analysis.

*Patients:* FSGS recurrent and non-recurrent patients were from the Kidney Transplantation Centre at Massachusetts General Hospital and at Jackson Memorial Hospital. Informed consent was obtained from the parents prior to recruiting and this study was approved by the respective Institutional Review Board.

*Serum suPAR determination*: Serum suPAR concentration was measured by Quantikine human uPAR immunoassay kit following the manufacture's protocol.

*Immunocytochemistry*: Podocytes were cultured as previously reported (Sever, S. et al. J Clin. Invest. 117,2095-2104 (2007)). After treatment, the fully differentiated podocytes were fixed and immunolabeled as described.

*Transmission electron microscopy (TEM):* TEM was performed according to the standard protocols (Sever, S. et al. J Clin. Invest. 117, 2095-2104 (2007)).

*Statistical analysis*: Statistical analyses were performed by using a Student paired or non-paired t-test. The null hypothesis was rejected at the 0.05 level. Values were presented as Mean ± SD.

### Results:

The role of the soluble form of uPAR in proteinuric kidney disease was investigated. The effects of purified suPAR protein on cultured podocytes was first examined. The fully differentiated mouse podocytes were treated with human suPAR for 24 h at 1-5 µg/ml and stained the cells with AP5, a proved antibody for active αᵥβ₃ integrin. In contrast to PBS treated control cells, which had negligible AP5 staining, cells treated with human suPAR protein had a strong AP5 staining on membrane, particularly on the leading edge (Figure 1A), indicating that suPAR protein activates αᵥβ₃ integrin, similar to membrane-anchored uPAR.

Next the effect of suPAR protein on proteinuria development was examined. suPAR protein was delivered into *Plaur* ^{-/-} mice through tail vein at 1 mg/kg body weight. Urine was collected before suPAR injection (0 h), 8 hand 24 h after injection and measured for total protein by Bradford's assay. As seen in Figure 1B, an increased level of urine protein was observed 8 h after suPAR injection, and by 24 h after suPAR protein delivery, mice developed marked proteinuria. In contrast, control mice which received the same amount of bovine serum albumin (BSA) did not develop any proteinuria (Figure 1B). To see whether any suPAR was deposited into kidney, the *Plaur* ^{*-*/*-*} mice were sacrificed and the kidney was taken out for cyrosection, and processed to immunohistochemistry. Extensive uPAR expression was found in kidneys treated with suPAR protein delivery. Double immunostaining with synaptopodin, a widely used podocyte marker, showed that suPAR was localized mostly in podocytes (Figure 1E). There was no uPAR expression or deposit in control kidneys treated with BSA (Figure 1E). These results evidence that delivery of suPAR protein could deposit into podocyte and more importantly it could induce proteinuria in *Plaur* ^{-/-} mice. The activity of αᵥβ₃ integrin was also examined in podocytes of suPAR protein delivered 32837mice by double immunostaining with AP5 and synaptopodin antibodies, and found it was significantly increased, as compared to that of BSA treated control, where only minimal active αᵥβ₃ integrin was observed in kidney, and even less in podocytes (Figure 1D). This finding is consistent with the above *in vitro* work, evidencing that suPAR, like its membrane form, activates αᵥβ₃ integrin, and then small GTPase, Rac, possibly through a pathway involving DOCK 180.

To further confirm the role of suPAR in proteinuria development and kidney disease, cross-kidney transplantation was performed and the effect of endogenous soluble form of uPAR was examined. The right kidney was excised from normal 57BL/6, and transplanted into *Plaur* ^{-/-} mice on the right side and *vice versa* (Figure 3A). Mice were randomly chosen to receive lipopolysaccharide (LPS) to induce proteinuria or PBS for control after recovery from surgery. For the 57BL/6 mice treated with LPS, significant foot process effacement was found in the native 57BL/6 kidney (Figure 1E), and more interestingly, massive foot process effacement was observed in the transplanted *Plaur* ^{-/-} kidney as well (Figure 1E). For the PBS treated 57BL/6 mice however, foot process effacement was not remarkable in either transplanted *Plaur* ^{*-*/*-*} kidney or native 57BL/6 kidney (Figure 1E). For the *Plaur* ^{*-*/*-*} mice treated with LPS, foot process effacement was readily observed in the transplanted 57BL/6 kidney but not in the native *Plaur* kidney (Figure 1F). In contrast, foot process effacement was not remarkable in either native *Plaur* ^{-/-} kidney or in the transplanted 57BL/6 kidney of PBS controlled mice (Figure 1F). These data put together evidence that the foot process effacement observed in the transplanted *Plaur* ^{-/-} kidney in the 57BL/6 mice treated with LPS was neither due to the surgery itself, nor due to direct LPS effect, but to some circulating extra-kidney factors. In order to determine whether uPAR is responsible for the phenomena, the sections were immunostained from both native and transplanted kidney with uPAR antibody. It was found that minimal uPAR expression in podocytes of PBS treated 57BL/6 kidney but not in those of PBS treated *Plaur* ^{-/-}kidney. With LPS treated 57BL/6 mice, increased podocyte uPAR expression was observed in the native 57BL/6 kidney, and interestingly, clear uPAR expression or deposit in the podocytes of transplanted *Plaur* ^{-/-} kidney was also observed (Figure 3B). With LPS treated *Plaur* ^{-/-} mice, increased uPAR expression was found in the transplanted 57BL/6 kidney, but not any uPAR in podocytes of native *Plaur* ^{-/-} kidney (Figure 3E). Combined with the electron microscopy analysis, these results indicate that in addition to membrane-bound uPAR, LPS could also induce generation of suPAR, which then goes to the kidney, deposit into the podocytes, and more importantly induce foot process effacement.

The last few decades witnessed much progress in podocyte biology, however, the exact pathogenesis underlying most proteinuric kidney diseases was heretofore, far from clear. This is true with the focal segmental glomerulosclerosis (FSGS). Though mutations to certain molecules including podocin, nephrin, α-actinin 4, TRPC6 have been claimed responsible for some familial form of FSGS, little was known about the pathogenesis of acquired form of FSGS. FSGS frequently leads to end-stage renal failure, and significantly recurring in 30-60% of transplanted kidneys. It was found herein, that suPAR could induce foot process effacement and proteinuria in 57BL/6 and *Plaur* ^{-/-} mice. This led to the next set of experiments to evaluate suPAR in FSGS patients. The serum level of suPAR of FSGS were measure in patients before transplantation and found that recurrent FSGS patients (*n* = 14, 5469 ±1837 pg/ml) had a significant higher level of suPAR than those non-recurrent patients (*n* = 4, 3586 ± 976 pg/ml, *P <* 0.022 versus recurrent), and than those normal subjects *(n* = 8, 1673 ± 395 pg/ml, *P <* 0.001 versus recurrent) (Figure 2A), evidencing that suPAR is a causative factor in recurrent FSGS. To further explore the effect of suPAR-rich FSGS sera, the sera were incubated with cultured podocytes and found that FSGS sera from recurrent patients but not sera from normal subjects, activated αᵥβ₃ integrin (Figure 2B), a finding observed with purified suPAR (Figure 1A).

*Summary*: suPAR could induce foot process effacement and proteinuria in 57BL/6 or *Plaur* ^{-/-} mice, through activating αᵥβ₃ integrin pathway. The level of suPAR is much higher in recurrent FSGS, compared to that in nonrecurrent FSGS and normal subjects. As with purified suPAR protein, suPAR-rich FSGS sera could activate αᵥβ₃ integrin. Considering the small molecular mass of suPAR, these findings evidence that suPAR is a FSGS recurrent factor. Since removing or blocking suPAR is possible, it warrants a promising strategy to enhance transplantation survival.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

The Abstract of the disclosure will allow the reader to quickly ascertain the nature of the technical disclosure. It is submitted with the understanding that it will not be used to interpret or limit the scope or meaning of the following claims.

The following numbered embodiments are also contemplated:
1. A method of treating renal diseases or disorders, comprising administering to a patient in need thereof, an effective amount of an agent which inhibits soluble urokinase receptor (suPAR) activity and/or function and/or modulates expression soluble and/or membrane bound urokinase receptor (uPAR) and/or pathways associated with urokinase receptor *in vivo* and, treating renal diseases or disorders.
2. The method of embodiment 1, wherein the renal diseases or disorders comprising:
   podocyte diseases or disorders, proteinuria, glomerular diseases, membranous glomerulonephritis, focal segmental glomerulonephritis, minimal change disease, nephrotic syndromes, pre-eclampsia, eclampsia, kidney lesions, collagen vascular diseases, stress, strenuous exercise, benign orthostatic (postural) proteinuria, focal segmental glomerulosclerosis (FSGS), IgA nephropathy, IgM nephropathy, membranoproliferative glomerulonephritis, membranous nephropathy, sarcoidosis, Alport's syndrome, diabetes mellitus, kidney damage due to drugs, Fabry's disease, infections, aminoaciduria, Fanconi syndrome, hypertensive nephrosclerosis, interstitial nephritis, Sickle cell disease, hemoglobinuria, multiple myeloma, myoglobinuria, diabetic nephropathy (DN), lupus nephritis, Wegener's Granulomatosis or Glycogen Storage Disease Type 1.
3. The method of embodiment 1, wherein an inhibitor of soluble and/or membrane bound urokinase receptor activity, expression and/or function comprises: nucleic acids, urokinase receptor mutants, oligonucleotides, polynucleotides, peptides, polypeptides, antibodies, small molecules, organic or inorganic molecules.
4. The method of embodiment 1, wherein a urokinase receptor mutant blocks the expression, function or activity of wild type soluble urokinase receptor molecules.
5. The method of embodiment 1 , wherein the soluble urokinase receptor inhibitor comprises an antibody, aptamer, antisense oligonucleotide, a natural agent, a synthetic agent or combinations thereof.
6. The method of embodiment 5, wherein an antisense oligonucleotide comprises: antisense RNA, antisense DNA, chimeric antisense oligonucleotides, antisense oligonucleotides comprising modified linkages, interference RNA (RNAi), short interfering RNA (siRNA); a micro, interfering RNA (miRNA); a small, temporal RNA (stRNA); or a short, hairpin RNA (shRNA); small RNA-induced gene activation (RNAa); small activating RNAs (saRNAs), or combinations thereof.
7. The method of embodiment 1, wherein an antibody specific for soluble urokinase receptor blocks or inhibits the function or activity of the soluble urokinase receptor.
8. The method of embodiment 1, wherein inhibition of soluble urokinase receptor activity, expression and /or function in associated pathways inhibits αᵥβ₃ integrin activation, GTPase, Rac and/or other associated molecules and pathways.
9. A method of identifying agents which modulate soluble urokinase receptor, function and/or activity *in vivo* comprising:
   contacting the soluble urokinase receptor with one or more agents;
   measuring physical or chemical changes in the soluble urokinase receptor molecule; and,
   identifying agents which modulate the uPAR-L expression, function and/or activity *in vivo.*
10. The method of embodiment 9, wherein a physical change comprising: binding to the suPAR molecule, conformational changes in a suPAR molecule, loss of binding activity to one or more molecules or combinations thereof.
11. The method of embodiment 9, wherein a chemical change comprising: modification of nucleic acids, modification of amino acids, splicing, fragmenting, glycosylation, degradation, bond formation, bond breaking or combinations thereof.
12. The method of embodiment 9, wherein the agent decreases suPAR activity or expression *in vivo* as compared to normal controls.
13. A method of identifying agents which modulate soluble urokinase receptor molecules (suPAR) expression, function and/or activity comprising:
   culturing a kidney cell or kidney cell line;
   contacting said cells with one or more agents;
   measuring the suPAR, expression, function, or activity; and,
   identifying agents which modulate soluble urokinase receptor molecules (suPAR), expression, function and/or activity.
14. The method of embodiment 13, wherein the soluble urokinase receptor molecule (suPAR) is inhibited by an agent by at least 10% as compared to a normal control.
15. The method of embodiment 10, wherein the soluble urokinase receptor molecule (suPAR) is inhibited by an agent by at least about 50% as compared to a normal control.
16. The method of embodiment 10, wherein the soluble urokinase receptor molecule (suPAR) is inhibited by an agent by 100% as compared to a control.
17. The method of embodiment 10, wherein the agent decreases soluble urokinase receptor molecule (suPAR) expression, function and/or activity by at least about 1 fold as compared to a normal control.
18. The method of embodiment 10, wherein the agent decreases soluble urokinase receptor molecule (suPAR) expression, function and/or activity by at least about 5 fold as compared to a normal control.
19. The method of embodiment 10, wherein the agent decreases soluble urokinase receptor molecule (suPAR) expression, function and/or activity up to 1000 fold as compared to a normal control.
20. A composition comprising a pharmaceutical composition and/or one or more soluble urokinase receptor molecule (suPAR) inhibitors and/or agents which inhibit decreases soluble urokinase receptor molecule (suPAR) expression, function, and/or activity in a therapeutically effective amount.
21. A biomarker for the diagnosis of a disease or disorder characterized by proteinuria and/or identification of individuals at risk of developing a disease or disorder characterized by proteinuria comprising: soluble or membrane bound urokinase receptor molecule (suPAR), variants, mutants or fragments thereof.
22. The biomarker of embodiment 21, wherein the identification of an individual at risk of developing disease or disorder characterized by proteinuria detects at least one biomarker or fragments thereof.
23. The biomarker of embodiment 21 , wherein the progression of disease or disorder characterized by proteinuria is correlated to an increase in soluble urokinase receptor (suPAR) molecules as compared to normal controls.
24. An antibody or aptamer specific for urokinase receptor (suPAR), mutants, variants, fragments, derivatives or analogs thereof.
25. An assay for measuring soluble urokinase receptor (suPAR) in patients comprising:
   contacting a biological sample from a patient with a detectably labeled anti-suPAR antibody; and, measuring soluble urokinase receptor (suPAR) in patients.
26. The assay of embodiment 25, wherein the soluble suPAR in a patient are correlated with disease progression comprising: podocyte diseases or disorders, proteinuria, glomerular diseases, membranous glomerulonephritis, focal segmental glomerulonephritis, minimal change disease, nephrotic syndromes, pre-eclampsia, eclampsia, kidney lesions, collagen vascular diseases, stress, strenuous exercise, benign orthostatic (postural) proteinuria, focal segmental glomerulosclerosis (FSGS), IgA nephropathy, IgM nephropathy, membranoproliferative glomerulonephritis, membranous nephropathy, sarcoidosis, Alport's syndrome, diabetes mellitus, kidney damage due to drugs, Fabry's disease, infections, aminoaciduria, Fanconi syndrome, hypertensive nephrosclerosis, interstitial nephritis, Sickle cell disease, hemoglobinuria, multiple myeloma, myoglobinuria, diabetic nephropathy (DN), lupus nephritis, Wegener's Granulomatosis or Glycogen Storage Disease Type 1.
27. A method of diagnosing a patient with kidney disease, comprising detecting in a patient or patient sample soluble uPAR; correlating the amounts of suPAR in the patient or patient sample with kidney disease or podocyte effacement; and,
   diagnosing a patient with kidney disease.
28. The method of embodiment 27, wherein the kidney disease comprises: podocyte diseases or disorders, proteinuria, glomerular diseases, membranous glomerulonephritis, focal segmental glomerulonephritis, minimal change disease, nephrotic syndromes, pre-eclampsia, eclampsia, kidney lesions, collagen vascular diseases, stress, strenuous exercise, benign orthostatic (postural) proteinuria, focal segmental glomerulosclerosis (FSGS), IgA nephropathy, IgM nephropathy, membranoproliferative glomerulonephritis, membranous nephropathy, sarcoidosis, Alport's syndrome, diabetes mellitus, kidney damage due to drugs, Fabry's disease, infections, aminoaciduria, Fanconi syndrome, hypertensive nephrosclerosis, interstitial nephritis, Sickle cell disease, hemoglobinuria, multiple myeloma, myoglobinuria, diabetic nephropathy (DN), lupus nephritis, Wegener's Granulomatosis or Glycogen Storage Disease Type 1.

## Claims

1. A composition for use in treating proteinuria in a renal disease or disorder **characterized by** proteinuria, the composition comprising an agent which blocks soluble urokinase receptor (suPAR) expression or activity.

2. The composition for use according to claim 1, wherein the agent comprises an antisense oligonucleotide, a polypeptide, or a small molecule.

3. The composition for use according to claim 1, wherein the agent is a mutant urokinase receptor (uPAR) polypeptide that blocks suPAR expression or activity.

4. The composition for use according to claim 1, wherein the agent is a suPAR specific antibody.

5. The composition for use according to claim 1, wherein the agent is an antisense oligonucleotide, the antisense oligonucleotide comprising: antisense RNA, antisense DNA, a chimeric antisense oligonucleotide, an antisense oligonucleotide comprising a modified linkage, interference RNA (RNAi), short interfering RNA (si RNA), microRNA (miRNA), small temporal RNA (stRNA), short hairpin RNA (shRNA), small RNA-induced gene activation (RNAa), small activating RNA (saRNA), or a combination thereof.

6. The composition for use according to any one of claims 1-5, which composition is a pharmaceutical composition comprising a carrier.

7. The composition for use according to any one of claims 1-6, wherein the agent inhibits αvβ3 integrin activation.

8. The composition for use according to any one of claims 1-7, wherein the composition comprises the agent in an amount effective to inhibit suPAR expression or activity by 10% or more as compared to a control.

9. The composition for use according to any one of claims 1-7, wherein the composition comprise the agent in an amount effective to inhibit suPAR expression or activity by 50% or more as compared to a control.

10. A composition for use in treating proteinuria in a renal disease or disorder **characterized by** proteinuria, the composition comprising an agent which binds to soluble urokinase receptor (suPAR) expression or activity.

11. The composition for use according to claim 10, wherein the agent comprises an antisense oligonucleotide, a polypeptide, or a small molecule.

12. The composition for use according to claim 10, wherein the agent is a mutant urokinase receptor (uPAR) polypeptide that blocks suPAR expression or activity.

13. The composition for use according to claim 10, wherein the agent is a suPAR specific antibody.

14. The composition for use according to any one of claims 10-13, which composition is a pharmaceutical composition comprising a carrier.

15. The composition for use according to any one of claims 1-14, wherein the renal disease or disorder **characterized by** proteinuria is: podocyte diseases or disorders, glomerular diseases, membranous glomerulonephritis, focal segmental glomerulonephritis, minimal change disease, nephrotic syndromes, pre-eclampsia, eclampsia, kidney lesions, collagen vascular diseases, stress, strenuous exercise, benign orthostatic (postural) proteinuria, focal segmental glomerulosclerosis (FSGS), IgA nephropathy, IgM nephropathy, membranoproliferative glomerulonephritis, membranous nephropathy, sarcoidosis, Alport's syndrome, diabetes mellitus, kidney damage due to drugs, Fabry's disease, infections, aminoaciduria, Fanconi syndrome, hypertensive nephrosclerosis, interstitial nephritis, Sickle cell disease, hemoglobinuria, multiple myeloma, myoglobinuria, diabetic nephropathy (DN), lupus nephritis, Wegener's Granulomatosis or Glycogen Storage Disease Type 1.
